# EUROPÄISCHE PATENTSCHRIFT

(11) **EP 2 365 372 B1**
(45) Veröffentlichungstag und Bekanntmachung des Hinweises auf die Patenterteilung: **27.07.2016**
(21) Anmeldenummer: 11157263.2
(22) Anmeldetag: 08.03.2011
(51) Int. Cl.: G02B 23/24, G02B 26/08, A61B 1/00, A61B 1/06

(54) **Schwenkprismenendoskop**
Swing prism endoscope
Endoscope à prismes oscillants

(30) Priorität: 10.03.2010 DE 102010010948
(43) Veröffentlichungstag der Anmeldung: 14.09.2011
(73) Patentinhaber: Karl Storz GmbH & Co. KG, 78532 Tuttlingen (DE)
(72) Erfinder: Baumann, Harald, 78532, Tuttlingen (DE)

(56) Entgegenhaltungen:
- WO-A1-99/66312
- DE-A1- 19 927 816
- US-A- 3 856 000
- US-A- 4 126 876
- US-A- 5 569 921
- US-A1- 2010 030 031

## Beschreibung

Die vorliegende Erfindung bezieht sich auf ein Schwenkprismenendoskop mit einer durch Schwenken eines Diamantprismas am distalen Ende des Endoskops einstellbaren Blickrichtung und auf ein Prisma für ein Schwenkprismenendoskop.

Neben Endoskopen für medizinische und nicht-medizinische technische Anwendungen, deren Blickrichtung parallel zur Längsachse des Schafts des Endoskops ist, wurden bereits früh Endoskope mit anderen feststehenden Blickrichtungen entwickelt. Mit der Blickrichtung eines Endoskops ist hier und im Folgenden immer die Richtung vom distalen Ende des Endoskops aus gemeint, in der ein Gegenstand liegt, der in der Mitte des mittels des Endoskops erfassten Bilds erscheint. Bei vielen Anwendungen ist jedoch eine feste Blickrichtung nachteilig. Im ungünstigsten Fall muss beispielsweise während eines medizinischen Eingriffs mehrfach das Endoskop gewechselt werden. In solchen Fällen ist die Verwendung eines Endoskops mit einer in situ einstellbaren bzw. verstellbaren Blickrichtung vorteilhaft.

Ein Schwenkprismenendoskop weist am distalen Ende ein schwenkbares Prisma auf, an dessen Grenzflächen in das Endoskop fallendes Licht gebrochen und reflektiert wird, bevor es beispielsweise mittels einer Stablinsenoptik zum proximalen Ende des Endoskops geleitet wird. Durch Schwenken des Prismas um eine Achse senkrecht zur Längsachse des Schafts des Endoskops ist die Blickrichtung einstellbar.

Herkömmliche Schwenkprismenendoskope weisen jedoch einen unbefriedigenden Blickrichtungsbereich auf. Wenn der Blickrichtungsbereich eine Blickrichtung parallel zur Achse des Schafts des Endoskops (0 Grad) umfassen soll, ist bislang nur ein kleiner Blickrichtungsbereich realisierbar, zwischen dessen extremen Blickrichtungen weniger als 45 Grad liegen. Ein großer Blickrichtungsbereich, zwischen dessen extremen Blickrichtungen 75 Grad oder mehr liegen, umfasst bislang bestenfalls beispielsweise Blickrichtungen von 45 Grad bis 120 Grad oder von 15 Grad bis 120 Grad oder von 10 Grad bis 115 Grad zur Achse des Schafts des Endoskops.

Eine Aufgabe besteht also darin, ein verbessertes Schwenkprismenendoskop, insbesondere ein Schwenkprismenendoskop mit einem größeren Blickrichtungsbereich, und ein verbessertes Prisma für ein Schwenkprismenendoskop zu schaffen, insbesondere ein Prisma, das in einem kleinen Raumbereich, dessen Ausdehnung beispielsweise durch die Kompatibilität mit herkömmlichen Trokaren und deren standardisierte Lumina begrenzt ist, einen größeren Blickrichtungsbereich ermöglicht, zu schaffen.

Das Patent US 4,126,876 schlägt vor, den Blickrichtungsbereich eines Dove Prismas zu verbessern, indem optische Materialien mit hohem Brechungsindex verwendet werden, wie z.B. Diamant. Hier ergibt sich allerdings die Gefahr von Doppelbrechungen. Überdies sind natürliche Diamanten sehr teuer und Reflexionen beeinträchtigen die optischen Eigenschaften.

Diese Probleme werden durch den Gegenstand des unabhängigen Anspruchs gelöst. Weiterbildungen sind in den.abhängigen Ansprüchen angegeben.

Ausführungsformen der vorliegenden Erfindung beruhen auf der Idee, das schwenkbare Prisma eines Schwenkprismenendoskops aus Diamant zu bilden. Diamant wird bislang in der Optik in der Medizintechnik lediglich dort eingesetzt, wo seine überragenden mechanischen, chemischen und thermischen Eigenschaften benötigt werden oder von Vorteil sind. Bei dünnen optisch transparenten Fenstern, die ein Fluid von Vakuum oder einem anderen Fluid trennen, und bei Beschichtungen werden die extreme Härte, die große Wärmeleitfähigkeit und die hohe Resistenz gegen viele Chemikalien genutzt. Als Material für optische Elemente wie Linsen oder Prismen wurde Diamant bislang in der Medizintechnik nicht in Betracht gezogen.

Gründe mögen die hohen Herstellungskosten, starke Restriktionen bei den erzielbaren geometrischen Parametern und - insbesondere in polykristallinem oder zahlreiche Fehlstellen aufweisendem Diamant beobachtete - Doppelbrechung gewesen sein.

Die vorliegende Erfindung beruht auch auf der Erkenntnis, dass das schwenkbare Prisma eines Schwenkprismenendoskops im Gegensatz zu optischen Elementen für viele andere Anwendungen ein vergleichsweise kleines Volumen aufweist. Das Prisma ist deshalb zum einen überhaupt und zum anderen zu angemessenen Kosten aus Diamant herstellbar.

Ein Vorteil eines schwenkbaren Prismas aus Diamant für ein Schwenkprismenendoskop besteht darin, dass das Prisma mit besonders kompakten Abmessungen realisiert werden kann. Ein weiterer Vorteil besteht darin, dass Diamant, insbesondere monokristalliner Diamant, eine hohe Transparenz für Licht im für das menschliche Auge sichtbaren Wellenlängenbereich aufweist. Ferner weist Diamant für sichtbares Licht eine vergleichsweise hohe Brechzahl und eine geringe Dispersion auf. Bei einer Herstellung des monokristallinen Diamants mittels chemischer Gasphasenabscheidung (engl.: Chemical Vapor Deposition - CVD) ist eine sehr geringe Doppelbrechung realisierbar.

Insgesamt ermöglicht die Verwendung von Diamant für ein schwenkbares Prisma eines Schwenkprismenendoskops einen Blickrichtungsbereich, der Blickrichtungen von 0 Grad (parallel zur Längsachse des Schafts des Endoskops) bis weit über 90 Grad, insbesondere bis 115 Grad oder 120 Grad, umfasst. Ein so großer Blickrichtungsbereich wurde bislang bei keinem anderen Schwenkprismenendoskop erreicht. Wie bereits eingangs erwähnt, ist die Blickrichtung die Richtung vom distalen Ende des Endoskops zu jenen Orten, die bei einer Betrachtung durch das Endoskop in der Mitte des Bildes erscheinen, unabhängig davon, ob dieses Bild unmittelbar durch ein Okular am proximalen Ende des Endoskops beobachtet oder mittels einer Kamera erfasst wird.

Ein Schwenkprismenendoskop mit einstellbarer Blickrichtung umfasst einen Schaft mit einem proximalen Ende und einem distalen Ende, einem Fenster (insbesondere einem gekrümmten Fenster) aus einem transparenten Material, das eine Öffnung am distalen Ende des Schafts fluiddicht verschließt, und ein schwenkbares Prisma am distalen Ende des Schafts zum einstellbaren Umlenken von Licht, das durch das Fenster in den Schaft des Schwenkprismenendoskops fällt, auf ein Objektiv, wobei das schwenkbare Prisma aus Diamant gebildet ist. Das schwenkbare Prisma ist erfindungsgemäß aus monokristallinem Diamant gebildet, der mittels chemischer Gasphasenabscheidung erzeugt ist.

Bei einem Schwenkprismenendoskop, wie es hier beschrieben ist, sind ferner erfindungsgemäß Antireflexbeschichtungen auf einer Lichteintrittsfläche und auf einer Lichtaustrittsfläche des schwenkbaren Prismas vorgesehen.

Ein Schwenkprismenendoskop, wie es hier beschrieben ist, kann einen Blickrichtungsbereich, innerhalb dessen die Blickrichtung des Schwenkprismenendoskops einstellbar ist, aufweisen, der eine axiale Richtung und eine dazu senkrechte Richtung umfasst. Die axiale Richtung ist die Richtung der Längsachse des Schafts des Schwenkprismenendoskops. Ein Schwenkprismenendoskop, wie es hier beschrieben ist, kann einen Blickrichtungsbereich mit einem Winkel von mindestens 110 Grad, insbesondere mindestens 120 Grad zwischen extremen Blickrichtungen umfassen. Dieser Blickrichtungsbereich kann eine Blickrichtung parallel zur Längsachse des Endoskops umfassen.

Ein Prisma für ein Schwenkprismenendoskop umfasst eine Lichteintrittsfläche, eine reflektierende Basisfläche und eine Lichtaustrittsfläche, wobei das Prisma aus Diamant gebildet ist, insbesondere aus monokristallinem Diamant. Das Prisma kann mittels chemischer Gasphasenabscheidung erzeugt sein. Ferner kann das Prisma Antireflexbeschichtungen auf der Lichteintrittsfläche und auf der Lichtaustrittsfläche aufweisen.

### Kurzbeschreibung der Figuren

Nachfolgend werden Ausführungsformen anhand der beigefügten Figuren näher erläutert. Es zeigen:
- Figur 1: eine schematische Darstellung eines Schwenkprismenendoskops;
- Figur 2: eine schematische Darstellung eines Prismas für ein Schwenkprismenendoskop;
- Figur 3: eine schematische Darstellung eines Schnitts durch das Prisma aus Figur 2;
- Figur 4: eine schematische Darstellung eines Schwenkprismas in zwei verschiedenen Positionen am distalen Ende eines Endoskops;
- Figur 5: eine schematische Darstellung eines Schwenkprismas in zwei verschiedenen Positionen am distalen Ende eines herkömmlichen Endoskops.

### Beschreibung der Ausführungsformen

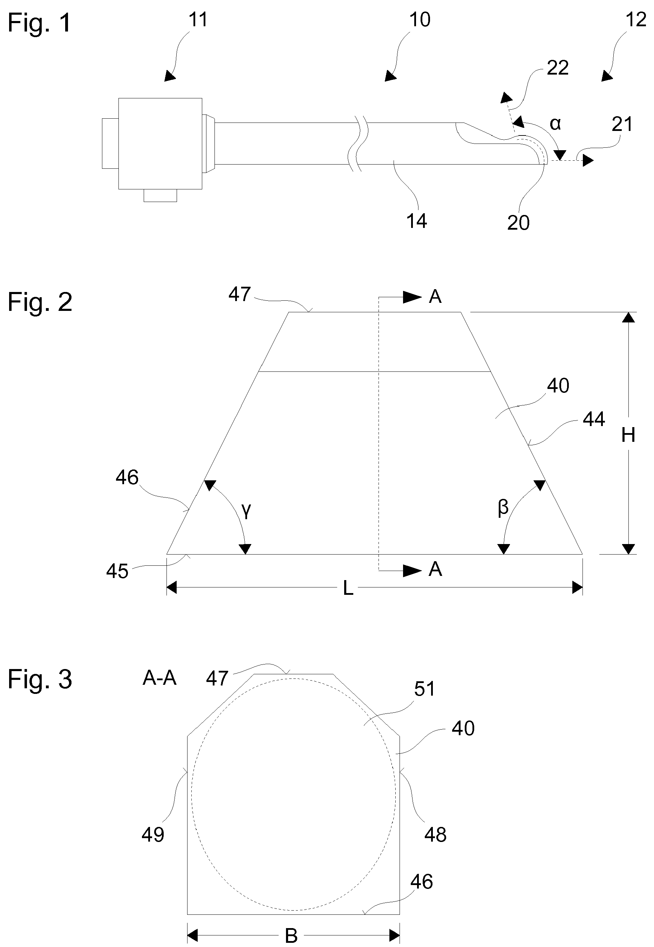

Figur 1 zeigt eine schematische Darstellung eines Endoskops 10 mit einem proximalen Ende 11 und einem distalen Ende 12. Auf das proximale Ende 11 des Endoskops 10 wird im Folgenden nicht näher eingegangen. Es kann ein Okular, eine Kupplung zu einem Lichtleitkabel zur Einkopplung von Licht von einer Lichtquelle zur Beleuchtung eines mittels des Endoskops zu betrachtenden Gegenstands, eine Kamera, eine Kupplung zu einem Lichtleitkabel zur Übertragung eines Bilds mittels eines geordneten Bündels von Lichtwellenleitern und/oder weitere Elemente umfassen, die in Figur 1 nicht dargestellt oder nur angedeutet sind.

Ein Schaft 14 erstreckt sich vom proximalen Ende 11 zum distalen Ende 12 des Endoskops 10. Mit den Bezugszeichen 11, 12 werden im Folgenden auch das proximale Ende bzw. das distale Ende des Endoskops 10 bezeichnet. Der Schaft 14 weist beispielsweise einen konstanten kreisförmigen Querschnitt auf. Am distalen Ende 12 weicht die Form des Schafts 14 von der kreiszylindrischen Form ab, wobei der Querschnitt des Schafts auch am distalen Ende 12 nicht über die von der überwiegenden kreiszylindrischen Form des Schafts 14 vorgegebene Kontur hinausragt. Der Schaft 14 ist insbesondere starr und weist eine Stablinsenoptik zur Übertragung eines Bilds vom distalen Ende 12 zum proximalen Ende 11 des Endoskops 10 auf. Alternativ ist der Schaft 14 flexibel. In diesem Fall enthält der Schaft 14 beispielsweise ein geordnetes Bündel von Lichtwellenleitern zur Übertragung eines Bilds vom distalen Ende 12 zum proximalen Ende 11 des Endoskops 10, oder eine Kamera ist nahe dem distale Ende 12 im Schaft 14 des Endoskops 10 angeordnet. Am distalen Ende 12 des Endoskops 10 ist ein gekrümmtes Fenster 20 aus einem transparenten Material vorgesehen. Das Fenster 20 verschließt fluiddicht, insbesondere hermetisch dicht, eine Öffnung am distalen Ende 12 des Endoskops 10. Das Fenster 20 weist beispielsweise die geometrische Gestalt eines Ausschnitts aus einem Kreiszylindermantel auf, wobei die Achse des Kreiszylinders senkrecht zur Zeichenebene der Figur 1 ist. Nahe dem Fenster 20 ist in dem Endoskop 10 ein Prisma angeordnet, das nachfolgend mit Bezug auf die Figuren 2 bis 4 näher beschrieben wird. Das bewegbare Prisma und das gekrümmte Fenster 20 ermöglichen eine Einstellung einer beliebigen Blickrichtung innerhalb eines Blickrichtungsbereichs. Wie bereits erwähnt, ist die Blickrichtung die Richtung vom distalen Ende 12 des Endoskops 10 aus, in der ein Gegenstand oder ein Ort liegt, der bei Betrachtung mittels des Endoskops 10 in der Mitte des mittels des Endoskops 10 gewonnen Bilds liegt. In Figur 1 sind zwei extreme Blickrichtungen 21, 22 angedeutet, die einen Winkel α einschließen. Neben dem Fenster 20 können eine oder mehrere Lichtaustrittsflächen angeordnet sein, durch die Licht zur Beleuchtung eines mittels des Endoskops 10 zu betrachtenden Gegenstands austreten kann.

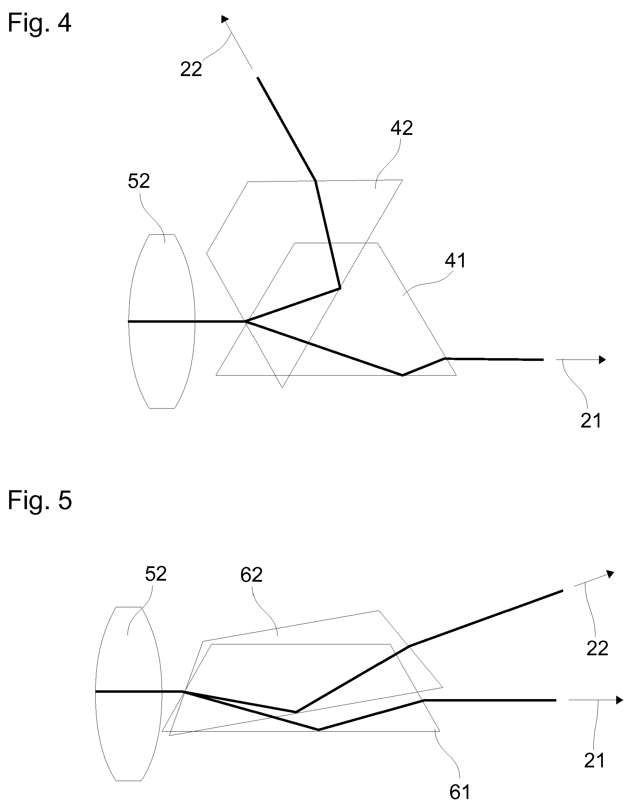

Die Figuren 2 und 3 zeigen schematische Darstellungen des bereits erwähnten Prismas 40 aus Diamant in dem erfindungsgemäßen Endoskop 10 nahe dem distalen Ende 12 des Endoskops 10. Die Zeichenebene der Figur 2 ist parallel zur Zeichenebene der Figur 1. Figur 3 zeigt einen Schnitt durch das Prisma 40 entlang der in Figur 2 angedeuteten Ebene A-A. Die Schwenkbarkeit des Prismas 40 und deren optische Wirkung werden unten mit Bezug auf die Figuren 4 und 5 dargestellt.

Das Prisma 40 weist eine Lichteintrittsfläche 44, eine reflektierende Basisfläche 45 und eine Lichtaustrittsfläche 46 auf, die jeweils senkrecht zur Zeichenebene der Figur 2 sind. Die Lichteintrittsfläche 44 und die Lichtaustrittsfläche 46 weisen erfindungsgemäß jeweils eine Antireflexbeschichtung auf. Die reflektierende Basisfläche 45 ist für einen hohen Reflexionsgrad verspiegelt. Eine der reflektierenden Basisfläche 45 gegenüberliegende Dachfläche 47 weist keine optische Funktion auf. Die Lichteintrittsfläche 44 und die Lichtaustrittsfläche 46 könnten sich in einer zur Zeichenebene der Figur 2 senkrechten Kante treffen. Dies hätte jedoch einen erhöhten Platzbedarf des Prismas 40 im Endoskop 10 zur Folge. Seitenflächen 48, 49 sind parallel zur Zeichenebene der Figur 2 und senkrecht zur Zeichenebene der Figur 3. In Figur 3 ist erkennbar, dass die Kanten zwischen der Dachfläche 47 und den Seitenflächen 48, 49 breite Fasen aufweisen können.

In Figur 3 ist ferner in unterbrochener Linie die Projektion 51 der Ränder desjenigen näherungsweise elliptischen Bereichs der Lichtaustrittsfläche 46 dargestellt, durch den Licht hindurchtritt, das zur Erzeugung eines Bilds durch das Endoskop 10 beiträgt. Der Abstand L zwischen den Kanten, in denen die Lichteintrittsfläche 44 bzw. die Lichtaustrittsfläche 46 an die reflektierende Basisfläche 45 grenzen, beträgt 5 mm bis 7 mm. Der Abstand H zwischen der reflektierenden Basisfläche 45 und der Dachfläche 47 des Prismas 40 beträgt 2,5 mm bis 3,5 mm. Der Abstand B zwischen den Seitenflächen 48, 49 beträgt 2 mm bis 3 mm. Der Winkel β zwischen der Lichteintrittsfläche 44 und der reflektierenden Basisfläche 45 und der Winkel γ zwischen der reflektierenden Basisfläche 45 und der Lichtaustrittsfläche 46 betragen jeweils zwischen 50 Grad und 70 Grad, insbesondere zwischen 55 Grad und 65 Grad. Die Winkel β, γ können gleich oder voneinander verschieden sein.

Die Figur 4 zeigt das oben anhand der Figuren 2 und 3 dargestellte Prisma 40 aus Diamant in zwei extremen Positionen 41, 42, zwischen denen das Prisma 40 beliebige eingestellt werden kann. Die Zeichenebene der Figur 4 ist parallel zu den Zeichenebenen der Figuren 1 und 2. Figur 4 zeigt ferner ein Objektiv 52 oder eine Linse zum Auffangen und Weiterleiten von Licht, das durch das oben anhand der Figur 1 dargestellte Fenster 20 hindurch in das Endoskop 10 fällt und vom Prisma 40 umgelenkt wird.

Für jede der beiden extremen Positionen 41, 42 des Prismas 40 ist jeweils ein Strahl dargestellt, der von einem in Blickrichtung 21, 22 liegenden und deshalb in der Mitte eines mittels des Endoskops 10 erfassten Bilds erscheinenden Gegenstands ausgeht. Der Winkel zwischen den beiden extremen Blickrichtungen 21, 22 beträgt bei diesem Beispiel näherungsweise 120 Grad, wobei die erste extreme Blickrichtung 21 parallel zur Achse des Schafts 14 des Endoskops 10 ist.

Die Figuren 5 entspricht der Figur 4, wobei jedoch ein Prisma in zwei extremen Positionen 61, 62 dargestellt ist, das nicht aus monokristallinem, mittels chemischer Gasphasenabscheidung hergestelltem Diamant, sondern aus einem Glas hergestellt ist. Dabei lässt eine der zwei extremen Positionen eine Blickrichtung in axialer Richtung zu.

Die beiden extremen Positionen 61, 62 sind durch den am distalen Ende 12 innerhalb des Endoskops 10 für das Prisma zur Verfügung stehenden Raumbereich festgelegt. Es ist erkennbar, dass das Prisma deutlich länger ist als das oben anhand der Figur 4 dargestellte erfindungsgemäße Prisma aus Diamant und deshalb nur um einen deutlich geringeren Winkel geschwenkt werden kann. In der Folge ist auch der Winkel zwischen den extremen Blickrichtungen 21, 22 deutlich geringer.

### Bezugszeichen

- 10: Endoskop
- 11: proximales Ende des Endoskops 10
- 12: distales Ende des Endoskops 10
- 14: Schaft des Endoskops 10
- 20: Fenster
- 21: erste extreme Blickrichtung
- 22: zweite extreme Blickrichtung
- 40: schwenkbares Prisma am distalen Ende 12 des Endoskops 10
- 41: Prisma 40 bei erster extremer Blickrichtung 21
- 42: Prisma 40 bei zweiter extremer Blickrichtung 22
- 44: Lichteintrittsfläche des Prismas 40
- 45: reflektierende Basisfläche des Prismas 40
- 46: Lichtaustrittsfläche des Prismas 40
- 47: Dachfläche des Prismas 40
- 48: Seitenfläche des Prismas 40
- 49: Seitenfläche des Prismas 40
- 51: Projektion des Rands eines Bereichs
- 52: Objektiv oder Linse
- 61: Glasprisma bei erster extremer Blickrichtung 21
- 62: Glasprisma bei zweiter extremer Blickrichtung 22

## Patentansprüche

1. **Schwenkprismenendoskop** (10) mit einstellbarer Blickrichtung, mit:
einem **Schaft** (14) mit einem proximalen Ende (11) und einem distalen Ende (12), einem **Fenster** (20) aus einem transparenten Material, das eine Öffnung am distalen Ende (12) des Schafts (14) fluiddicht **verschließt;**
einem **Prisma** (40), das schwenkbar ist und am distalen Ende (12) des Schafts (14) angeordnet ist, zum einstellbaren Umlenken von Licht, das durch das Fenster (20) in den Schaft (14) des Schwenkprismenendoskops (10) fällt, auf ein Objektiv (52), wobei das Prisma
eine Lichteintrittsfläche (44), eine reflektierenden Basisfläche (45) und eine Lichtaustrittsfläche (46) umfasst,
**gekennzeichnet dadurch, dass**
das Prisma (40) aus **monokristallinem** Diamant gebildet ist, mittels **chemischer Gasphasenabscheidung** gebildet ist und ferner **Antireflexbeschichtungen** auf einer Lichteintrittsfläche (44) und auf einer Lichtaustrittsfläche (46) des Prismas (40) aufweist.

2. Schwenkprismenendoskop (10) nach Anspruch 1, bei dem ein Blickrichtungsbereich, innerhalb dessen die Blickrichtung des Schwenkprismenendoskops einstellbar ist, eine **axiale** Richtung und eine dazu **senkrechte** Richtung umfasst.

3. Schwenkprismenendoskop (10) nach einem der vorangehenden Ansprüche, bei dem der Blickrichtungsbereich einen Winkel von mindestens **110 Grad** umfasst.

## Claims

1. Swing prism endoscope (10) with an adjustable viewing direction, comprising:
a shank (14) with a proximal end (11) and a distal end (12),
a window (20) made of a transparent material which seals an opening at the distal end (12) of the shank (14) in a fluid-tight manner;
a prism (40) which is swivelable and arranged at the distal end (12) of the shank (14), for adjustably deflecting light, which enters into the shank (14) of the swing prism endoscope (10) through the window (20), onto an objective (52), wherein the prism comprises a light-entrance area (44), a reflecting base area (45) and a light-exit area (46),
**characterized in that**
the prism (40) is made of monocrystalline diamond, it is formed by means of chemical vapour deposition and, furthermore, it has antireflection coatings on a light-entrance area (44) and on a light-exit area (46) of the prism (40).

2. Swing prism endoscope (10) according to Claim 1, in which a viewing direction range, within which the viewing direction of the swing prism endoscope is adjustable, comprises an axial direction and a direction perpendicular thereto.

3. Swing prism endoscope (10) according to one of the preceding claims, wherein the viewing direction range comprises an angle of at least 110 degrees.

## Revendications

1. Endoscope à prisme oscillant (10) avec une direction d'observation ajustable, comprenant :
une tige (14) avec une extrémité proximale (11) et une extrémité distale (12),
une fenêtre (20) constituée d'un matériau transparent, qui ferme de manière étanche aux fluides une ouverture au niveau de l'extrémité distale (12) de la tige (14) ;
un prisme (40) qui peut pivoter et est disposé au niveau de l'extrémité distale (12) de la tige (14) pour effectuer une déviation ajustable de la lumière qui, à travers la fenêtre (20) dans la tige (14) de l'endoscope à prisme oscillant (10), tombe sur un objectif (52), le prisme comprenant une surface d'entrée de la lumière (44), une surface de base réfléchissante (45) et une surface de sortie de la lumière (46),
**caractérisé en ce que**
le prisme (40) est formé de diamant monocristallin, est formé par dépôt chimique en phase gazeuse et présente en outre des revêtements antiréfléchissants sur une surface d'entrée de la lumière (44) et sur une surface de sortie de la lumière (46) du prisme (40).

2. Endoscope à prisme oscillant (10) selon la revendication 1, dans lequel une région de direction d'observation à l'intérieur de laquelle la direction d'observation de l'endoscope à prisme oscillant peut être ajustée comprend une direction axiale et une direction perpendiculaire à celle-ci.

3. Endoscope à prisme oscillant (10) selon l'une quelconque des revendications précédentes, dans lequel la région de direction d'observation comprend un angle d'au moins 110 degrés.
